Europäisches Patentamt

European Patent Office

Office européen des brevets

(11) Publication number: **0 241 335 B1**

# EUROPEAN PATENT SPECIFICATION

(45) Date of publication of patent specification: **03.06.92**

(51) Int. Cl.⁵: **C07C 21/19**, C07C 17/28

(21) Application number: **87400592.9**

(22) Date of filing: **17.03.87**

(54) 1-substituted conjugated alka-(E,Z)-diene compounds and a method for the preparation thereof.

(30) Priority: **19.03.86 JP 61885/86**

(43) Date of publication of application:
**14.10.87 Bulletin 87/42**

(45) Publication of the grant of the patent:
**03.06.92 Bulletin 92/23**

(84) Designated Contracting States:
**DE ES FR GR IT**

(56) References cited:
**DE-A- 2 916 068**
**GB-A- 1 601 346**
**US-A- 3 845 108**

**REGISTRY HANDBOOK, CHEMICAL AB-STRACTS SERVICE, 1979, Columbus, Ohio, USA; Registry numbers 70 831-26-4 through 72 467-41-5, page 2157RH**

**TETRAHEDRON, vol. 33, 1977; CLIVE A. HEN-DRICK, "The synthesis of insect sex pheromones", pp. 1845-1889**

(73) Proprietor: **Shin-Etsu Chemical Co., Ltd.**
**6-1, Otemachi 2-chome**
**Chiyoda-ku Tokyo 100(JP)**

(72) Inventor: **Yamamoto, Akira**
**2-14-45, Gochi**
**Joetsu-shi, Niigata-ken(JP)**
Inventor: **Fukumoto, Takehiko**
**Shin-Etsu Kagaku Kirinoki Ryo 27-20, Sanai-cho**
**Joetsu-shi, Niigata-ken(JP)**

(74) Representative: **Armengaud Ainé, Alain et al**
**Cabinet ARMENGAUD AINE 3 Avenue Bugeaud**
**F-75116 Paris(FR)**

## Description

The present invention relates to 1-substituted conjugated alka-(E,Z)-dienes as a class of novel compounds or, more particularly, to 1-halo-(E,Z)-7,9-dodecadienes and a method for the preparation thereof.

1-Substituted conjugated alka-(E,Z)-dienes such as 1-halo-(E,Z)-7,9-alkadienes belong to a class of novel compounds not known in the prior art or not described in any published literatures. The compounds are useful as an intermediate for the synthesis of various kinds of conjugated diene compounds including very important compounds having activity as a sex pheromone of insects, having fragrance as a synthetic perfume and so on.

As is well known, the so-called Wittig reaction is the most widely and effectively practiced method for the synthesis of an unsaturated diene compound having conjugated double bonds. When the Wittig reaction is applied to the synthesis of a special diene compound with conjugated double bonds, such as the sex pheromone compounds of insects which are mostly in the form of an acetate, alcohol, and aldehyde of the conjugated diene compound, it is essential that the diene compound should have a functional group such as a hydroxy group, halogen atom at one molecular end even after completion of the Wittig reaction. In this regard, the starting compound for the Wittig reaction should be difunctional either at the end of the oxo group or at the phosphoranylidene group to pertain to the Wittig reaction.

It is a usual way that the Wittig reaction is undertaken with a starting compound of which either the oxo group or the phosphoranylidene group is protected with a hydroxy group or a hydroxy group in an etherified form. This method, however, is not quite advantageous industrially because the synthetic procedure for the desired compound includes the troublesome steps for providing the terminal protecting group and elimination thereof after the reaction, if not to mention the expensiveness of the specific reagent used for the reaction. Alternatively, an attempt has been made to prepare a conjugated (E,Z)-diene compound by the cis-hydrogenation of the triple bond in an ene-yne compound although this method is also disadvantageous in respect of the expensiveness of the hydrogenated borane compound used as a reagent in the reaction.

Turning now to the problem of controlling the population of insect pests in agriculture and forestry by means of a sex pheromone of the respective insect species. Many sex pheromone compounds belong to the class of the conjugated (E,Z)-diene compounds as is exemplified by E,Z-7,9-dodecadienyl acetate which is the sex pheromone compound of European grape vine moths so that it is eagerly desired to develop an efficient route for the synthetic preparation of such a conjugated diene compound.

When the Wittig reaction is applied to the synthesis of a conjugated diene compound having a terminal structure of acetate, alcohol or aldehyde as is the case in the above mentioned E,Z,-7,9-dodecadienyl acetate of the formula

$$CH_3CH_2CH = {}^{(Z)}CHCH = {}^{(E)}CH(CH_2)_6OCOCH_3,$$

either the oxo end or the phosphoranylidene end of the reactant compound must be difunctional. For example, the specification of U.S.Patent No. 3,845,108 teaches a process of the Wittig reaction for the synthesis of the compound utilizing an aldehyde synthesized by the use of the anion of diethyl 2-(cyclohexylimino)ethyl phosphonate following oxidation of methyl 7-bromoheptanoate. A similar synthetic route is disclosed in Tetrahedron, volume 33, page 1845 (1977) for the preparation of the same compound in which the hydroxy group in 7-bromoheptyl alcohol is protected by replacing the hydrogen atom with a tetrahydropyranyl group to form an ether linkage. These prior art methods, however, are disadvantageous and not practical in the industrial production of the compound in respect of the little availability and expensiveness of the starting reactants.

In view of the above described problems, the inventors have conducted extensive investigations and synthesized a dialkyl acetal of a 9-halo-2-nonyn-1-al by a synthetic route in which a 8-halo-1-octyne as the starting material is first converted into a 8-halo-1-octyn-1-yl magnesium halide by use of a Grignard reagent followed by the reaction with trimethyl or triethyl orthoformate.

Thereafter, this acetal compound is hydrogenated by use of a hydrogenation catalyst such as P-2Ni followed by hydrolysis in an aqueous solution of hydrogen chloride to produce 9-halo-(E)-2-nonen-1-al quite easily.

Further, a phosphonium salt represented by the general formula $R^3CH_2PR^4{}_3.X^1$, in which $R^3$ is a hydrogen atom or an alkyl group having 1 to 15 carbon atoms, $R^4$ is a phenyl group or an t-Butyl group and $X^1$ is a halogen atom, is first converted into a phosphoranylidene compound by use of various kinds of base compounds and the phosphoranylidene compound is subjected to the Wittig reaction with the 9-halo-(E)-2-nonen-1-al mentioned above to give a 1-halo-(E,Z)-7,9-alkadiene in a good yield according to the following reaction route:

$$CH \equiv C(CH_2)_6X^2$$
$$\rightarrow (R^2O)_2CHC \equiv C(CH_2)_6X^2$$
$$\rightarrow OHCCH = CH(CH_2)_6X^2$$

→ $R^3CH = {}^{(Z)}CHCH = {}^{(E)}CH(CH_2)_6 X^2$.

This synthetic route is advantageous in respect of the compatibility with a production of the compound in an industrial scale because the terminal halogen atom $X^2$ does not pertain to any step of the reaction sequence to be retained as the functional group in the product compound. Therefore, this procedure can be advantageously applied to the preparation of an intermediate for the synthesis of, for example, E,Z-7,9-dodecadienyl acetate as the sex pheromone compound of European grape vine moths and E,Z-10,12-hexadecadien-1-ol as the sex pheromone compound of silkworm moths.

Thus, the present invention provides a method of the synthetic preparation of 1-halo-substituted conjugated alka-(E,Z)-diene compound or, in particular, a 1-halo-(E,Z)-7,9-dodecadiene represented by the general formula

$$R^3CH = {}^{(Z)}CHCH = {}^{(E)}CH(CH_2)_6 X, \qquad (I)$$

in which X is an atom of chlorine or bromine, and $R^3$ is a hydrogen atom or an alkyl group having 1 to 15 carbon atoms which comprises a Wittig reaction of a 9-halo-(E)-2-monen-1-al of the formula

$$X-(CH_2)_6 -CH = {}^{(E)}CHCHO,$$

in which X has the same meaning as defined above, with a phosphoranylidenation product of a phosphonium salt of the formula

$$R^3CH_2 PR_3 X^1,$$

in which R is a phenyl group or an alkyl group having 1 to 10 carbon atoms and $X^1$ is an atom of a halogen selected from the class consisting of chlorine, bromine and iodine.

Following is a more detailed description of the synthetic procedure of the 1-halo-(E,Z)-7,9-dodecadiene compound according to the inventive method.

In the first place, a 8-halo-1-octyne of the formula $Z^2-(CH_2)_6 C≡CH$ is reacted with a Grignard reagent of the formula $R^1MgX$, in which $R^1$ is a methyl or ethyl group and X is an atom of chlorine or bromine, in tetrahydrofuran at a temperature in the range from 40 to 80 °C to form a 8-halo-1-octyn-1-yl magnesium halide. Then, a trialkyl orthoformate of the formula $(R^1O)_3 CH$ is added dropwise to the reaction mixture kept at 80 to 100 °C and the reaction mixture is heated for about 20 hours so that a dialkyl acetal of a 9-halo-2-nonyn-1-al is obtained in a high yield.

In the second place, the thus obtained acetal compound is hydrogenated and hydrolyzed so that a 9-halo-(E)-2-nonen-1-al of the formula $X^2-(CH_2)-{}_6 CH = {}^{(E)}CHCHO$ is easily obtained. Namely, the

$\alpha,\beta$-unsaturated aldehyde compound can be obtained without affecting the halogen atom at the $\omega$-position. The hydrogenation catalyst used here is preferably a Lindler catalyst, palladium catalyst, for example, supported on a carbon carrier or a nickel catalyst such as P-2Ni. The reaction conditions for the hydrogenation preferably include a hydrogen pressure in the range from 0.5 to 10 $kg/cm^2G$ and a temperature in the range from 0 to 70 °C. The hydrolysis reaction is preformed by adding a 20% hydrochloric acid into an organic solution of the reaction mixture prepared by using a water-immiscible organic solvent such as aliphatic and aromatic hydrocarbon solvents, e.g. n-hexane and toluene, and chlorinated hydrocarbon solvents, e.g. methylene chloride. Although the use of methylene chloride is preferable in respect of the extremely high reaction velocity of the hydrolysis, the hydrocarbon solvents should be used in view of the easiness in the recovery and recycling of the solvent. The amount of the 20% hydrochloric acid should be in the range from 0.5 to 2.0 times by volume of the acetal compound. The temperature for the hydrolysis should be in the range from 0 to 40 °C while a higher temperature is undesirable due to the increased amount of by-products.

In the third place, the phosphonium salt compound used in the Wittig reaction is a compound represented by the general formula $CH_3 CH_2 CH_2 PR_3 X^1$. The halogen $X_1$ may be a chlorine, bromine or iodine atom and $R^4$ is either aryl such as phenyl which can be optionally, nucleus-substituted with an alkyl group (such as methyl, ethyl) or an hydroxy or alkoxy group, or halogen atom or a trialkyl phosphine such as tri (tertbutyl) phosphine.

The reaction is performed by dissolving these reactions in an organic solvent and hearing the solution, preferably, at 20 to 200 °C under anhydrous conditions Suitable organic solvents include nitriles, e.g. acetonitrile, aromatic hydrocarbons, e.g. benzene, toluene and xylene, and tetrahydrofuran.

The base compound used for converting the phosphonium salt into a phosphoranylidene compound can be any of those forming carbanions such as n-butyl lithium and those forming alkoxy anions such as potassium tert-butoxide. Suitable solvents, which should be used in an anhydrous condition, include benzene, toluene, hexane, tetrahydrofuran, dimethyl formamide, dimethyl sulfoxide, diethyl ether, glyme and the like.

The reaction of the thus prepared phosphoranylidene compound and the above mentioned aldehyde compound can be performed in the same solution as used in the preparation of the phosphoranylidene compound. The reaction temperature is in the range from -78 °C to +80 °C so that the reaction mixture should be chilled or heated according to need. The reaction is complete within several minutes to 3 hours or, in most cases, 30 minutes to 1 hour. The reaction should be performed under a stream of an inert gas such as nitrogen, argon and the like.

The thus obtained crude reaction mixture is subjected to distillation under reduced pressure, column chromatography, thin-layer chromatography and other known procedures to isolate and purify the desired product.

In respect of the purity of the geometric isomers of the desire 1-halo-(E,Z)-7,9-dodecadiene compound obtained in the above described method, the E-type steric configuration at the 7-position is complete while, relative to the steric configuration at the 9-position, the product usually contains 10 to 30% of 1-halo-(E,E)-7,9-dodecadiene isomer. This disadvantage can be at least partly overcome by performing the reaction under the so-called "salt-free" condition taught in Journal of American Chemical Society, volume 104, page 5821 (1982) for the preferential formation of the cis-isomer or other means for controlling steric specificity. For example, no particular difficulty is encountered in obtaining 1-halo-(E,Z)-7,9-dodecadiene and (E,Z)-7,9-dodecadienyl acetate derived therefrom as the sex pheromone of European grape vine moths having 75% or higher purity relative to the geometric isomers. The above mentioned 1-halo-(E,Z)-7,9-dodecadiene is a novel compound not described in any prior art literatures.

In the following, the method of the present invention is described in more detail by way of examples for the preparation of 1-chloro- and 1-bromo-(E,Z)-7,9-dodecadienes although the invention is by no means limited to the preparation of these particular compounds.

Example 1.

A diethyl acetal of 9-chloro-2-nonyn-1-al was synthesized in the following manner. In the first place, a Grignard reagent methyl magnesium chloride in an amount corresponding to 2.1 moles was prepared in 400 ml of tetrahydrofuran under an atmosphere of nitrogen. Thereafter, 289 g of 8-chloro-1-octyne were added dropwise into the Grignard solution kept at 50 to 60 °C and the reaction mixture was heated under agitation at 70 °C for 1 hour. The reaction mixture was then admixed with 200 ml of toluene and further 325 g of triethyl orthoformate $(C_2H_5O)_3CH$ were added dropwise into the reaction mixture kept at 80 to 90 °C followed by further continued agitation of the mixture at 90 to 100 °C for about 20 hours to complete the reaction. After completion of the reaction, the reaction mixture was poured into a large volume of an aqueous solution of ammonium chloride and the organic phase taken by phase separation was stripped of the solvent and distilled under reduced pressure to give 250 g of a fraction boiling at 150 to 160 °C under a pressure of 3 mmHg which could be identified to be the diethyl acetal of 9-chloro-2-nonyn-1-al having a purity of at least 96%.

9-Chloro-(E)-2-nonen-1-al was then prepared by the hydrogenation of the above obtained diethyl acetal of 9-chloro-2-nonyn-1-al followed by hydrolysis in the following manner. Thus, a hydrogenation catalyst P-2Ni in an amount corresponding to 0.03 mole was prepared in 300 ml of ethyl alcohol, to which 1 g of ethylene diamine and 250 g of the diethyl acetal of 9-chloro-2-nonyn-1-al were added. The hydrogenation reaction of this acetal compound was performed in an autoclave at a temperature of 20 to 40 °C under a hydrogen pressure of 5 kg/cm²G until the results of gas chromatographic analysis indicated complete disappearance of the starting acetal compound in the reaction mixture. Thereafter, the reaction mixture taken out of the autoclave was admixed with each 300 ml of water and n-hexane followed by phase separation. The organic phase was stripped of n-hexane and then admixed with 400 ml of methylene chloride and 300 ml of 20% hydrochloric acid to effect the hydrolysis of the acetal compound at 0 to 30 °C until the results of the gas chromatographic analysis indicated complete absence of the acetal compound in the reaction mixture. The reaction mixture was washed with a saturated aqueous solution of sodium chloride containing 2% of sodium carbonate and stripped of the methylene chloride by distillation under reduced pressure.

1-Chloro-(E,Z)-7,9-dodecadiene was prepared from the above obtained 9-chloro-(E)-2-nonen-1-al by the Wittig reaction in the following manner. Thus, 76 g of potassium tert-butoxide were gradually added to a suspension of 270 g of n-propyl triphenyl phosphonium bromide in 500 ml of anhydrous tetrahydrofuran so that the phosphonium salt compound was converted to the phosphoranylidene compound of the formula $CH_3CH_2CH=P(C_6H_5)_3$. The reaction mixture turned dark red. After agitation of the mixture for about 30 minutes, 122 g of the 9-chloro-(E)-2-nonen-1-al were added dropwise into the mixture kept at 15 to 20 °C followed by further continued agitation for 1 hour at 20 °C. Thereafter, the reaction mixture was stripped of tetrahydrofuran by distillation under reduced pressure and admixed with each 200 ml of water and n-hexane. The precipitates of triphenyl phosphine oxide formed in the mixture were removed by filtration. The filtrate was subjected to phase separation and the organic phase was stripped of n-hexane and then distilled under reduced pressure to give 85 g of a product which could be identified to be 1-chloro-7,9-dodecadiene having a purity of at least 75% relative to 1-chloro-(E,Z)-7,9-dodecadiene from the analytical results shown below by the mass spectrometric, NMR spectrometric and infrared absorption spectrometric analyses.

Mass-spectrometric data: m/e (relative intensity) 39(16);41(34);55(30)67(100);68(42);81(50);82(78); 95(83);109(17);123(68);144*(12);167*(10);171*(10); 200*(M$^+$,34)

(The peaks of the asterisked m/e number were each accompanied by another peak ascribable to the $^{37}$Cl isotope.)

NMR data: δ, ppm

CH$_3$CH$_2$CH$_a$ = CH$_b$CH$_c$ = CH$_d$CH$_2$(CH$_2$)$_4$CH$_2$Cl

1.0 (t, 3H,J = 7Hz);1.20-1.75(broad,8H); 2.25(m,4H,-CH$_2$CH = CH-);3.50(t,2H,J = 6.5 Hz,-CH$_2$Cl); 5.20 (td, 1$\overline{H}$,-H$_a$,J = 7.5Hz);5.50(td,1H,-H$_d$,J-7H$\overline{z}$); 5.80 (dd,1H,-H$_b$,J[H$_a$-H$_b$] = 10.5Hz); 6.25 (dd,1H,-H$_c$,J[H$_c$-H$_b$] = 15Hz,J[H$_c$-H$_b$] = 6.5Hz)

Infrared absorption bands: cm$^{-1}$ (film method) 3020;2960;2925;2875;2850;1690;1650;1470;1415;1-380; 980;945;730

A 150 g portion of the thus obtained 1-chloro-(E,Z)-7,9-dodecadiene was admixed with 150 g of anhydrous potassium acetate and 100 g of acetic acid and the mixture was heated at 160 °C for 6 hours under agitation in an atmosphere of nitrogen to convert the chloride into acetate. After completion of the reaction, the reaction mixture was poured into a large volume of water and the organic phase taken by phase separation was distilled under reduced pressure to give 80 g of 7,9-dodecadienyl acetate of which the purity of the E,Z-isomer was at least 75%.

Example 2.

1-Bromo-(E,Z)-7,9-dodecadiene was prepared by the following synthetic route. In the first place, 292 g of the diethyl acetal of 9-bromo-2-nonyn-1-al were prepared in the same manner as in the preparation of 1-chloro-2-nonyn-1-al in Example 1 excepting replacement of 289 g of 8-chloro-1-octyne with 378 g of 8-bromo-1-octyne. The thus obtained acetal compound had a boiling point of 158 to 165 °C under a pressure of 3 mmHg and the purity thereof was at least 90%.

9-Bromo-(E)-2-nonen-1-al was prepared in the same manner as in the preparation of 9-chloro-(E)-2-nonen-1-al from the diethyl acetal of 9-chloro-2-nonyn-1-al in Example 1 excepting replacement of 250 g of the diethyl acetal of 9-chloro-2-nonyn-1-al with 292 g of the diethyl acetal of 9-bromo-2-nonyn-1-al. The reaction product was used as such as the starting material of the next step without further purification.

1-Bromo-(E,Z)-7,9-dodecadiene was prepared from 9-bromo-(E)-2-nonen-1-al in the same manner as in the preparation of 1-chloro-(E,Z)-7,9-dodecadiene from 9-chloro(E)-2-nonen-1-al in Example 1 excepting replacement of 9-chloro-(E)-2-nonen-1-al with 9-bromo-(E)-2-nonen-1-al prepared in the above described manner. The yield of the product was 90 g and the compound could be identified to be the desired 1-bromo-(E,Z)-7,9-dodecadiene from the analytical data shown below.

Mass-spectrometric data:m/e(relative intensity) 39(12);41(29);55(29)67(100);68(38);81(53);82(83); 95(96);109(21);123(10);135*(5);188*(8);215*(3); 244*-(M$^+$,24)

(The peaks of the asterisked m/e number were each accompanied by a peak ascribable to the $^{81}$Br isotope.)

NMR data: δ, ppm

CH$_3$CH$_2$CH$_a$ = CH$_b$CH$_c$ = CH$_d$CH$_2$(CH$_2$)$_4$CH$_2$Br

1.0(t,3H,J = 7 Hz);1.20-1.75(broad,8H); 2.25(m,4H,-CH$_2$CH = CH-);3.40(t,2H,J = 5 Hz,-CH$_2$Br); 5.20-(t$\overline{d}$,1H,-H$_a$,J = 7.5 Hz);5.45(td,1H,-H$_d$,$\overline{J}$ = 7Hz); 5.80-(dd,1H,-H$_b$,J[H$_a$-H$_d$] = 10Hz); 6.20(dd,1H,-H$_c$,J[H$_c$-H$_d$] = 15Hz,J[H$_c$-H$_b$] = 6Hz)

Infrared absorption bands: cm$^{-1}$ (film method) 3020;2960;2925;2875;2850;1695;1650;1470;1415;1-380; 980;945;720

The same procedure for the preparation of E,Z-7,9-dodecadienyl acetate as in Example 1 was repeated excepting replacement of 85 g of 1-chloro-(E,Z)-7,9-dodecadiene with 90 g of 1-bromo-(E,Z)-7,9-dodecadiene to give 62 g of the desired product having a purity of at least 75% relative to the content of the (E,Z)-isomer.

**Claims**

1. A process for the synthetic preparation of a 1-halo-substituted conjugated alka-(E,Z)-diene compound represented by the general formula :

R$^3$CH = $^{(Z)}$CHCH = $^{(E)}$CH(CH$_2$)$_6$X

in which X is an atom of chlorine or bromine, and R$^3$ is a hydrogen atom or an alkyl group having 1 to 15 carbon atoms which comprises :
- performing a Wittig reaction of a 9-halo-(E)-2-nonen-1-al of formula

X-(CH$_2$)$_6$-CH = CH-CHO,

X representing Cl or Br,
with a phosphoranylidenation product of a phosphonium salt of the formula

R$^3$CH$_2$P(R)$_3$X' ,

in which R is a phenyl group or a tertiarybutyl group and X' is an halogen atom selected from the class consisting of chlorine, bromine and iodine.

2. Process according to claim 1, wherein $R^3$ represents a $-CH_2-CH_3$ group.

3. A modification of the process according to claim 1 or 2, wherein said 9-halo-(E)-2-nonen-1-al is obtained by :
   - reacting 8-halo-1-octyne with a Grignard reagent to obtain a 8-halo-1-octyn-1-yl magnesium halide,
   - reacting said 8-halo-1-octyn-1-yl magnesium halide with an alkyl orthoformate to obtain the corresponding acetal, and
   - hydrogenating and hydrolyzing the acetal compound to obtain said 9-halo-(E)-2-nonen-1-al.

4. A process for the synthetic preparation of sex pheromone compounds of insects or fragrances of the class of the conjugated (E,Z)-diene compounds, particularly with an acetate, alcohol or aldehyde end unit, such as E,Z,-7,9-dodecadienyl acetate and E,Z-10,12 hexadecadien-1-ol comprising a modification of the process of claim 1, wherein the halogen is replaced by an acetate, an hydroxy, or an aldehyde group.

5. 1-halo-substituted conjugated alka-(E,Z)-diene compound having the general formula.

$$R^3CH = {}^{(Z)}CHCH = {}^{(E)}CH(CH_2)_6X$$

in which X is an atom of chlorine or bromine, and $R^3$ is an alkyl group having 1 to 15 carbon atoms, particularly a $CH_3-CH_2$ group

**Revendications**

1. Procédé de synthèse de composés alca-(E,Z)-diéniques conjugués, substitués par un halogène en position 1, représentés par la formule générale

$$R^3CH = (Z)CHCH = (E)CH(CH2)_6X$$

dans laquelle X est un atome de chlore ou de brome, et $R^3$ est un atome d'hydrogène ou un groupe alkyle ayant 1 à 15 atomes de carbone,
procédé comprenant :
   - la réalisation d'une réaction de Wittig entre un 9-halo-(E)-2-nonen-1-al de formule

$$X-(CH_2)_6-CH = CH-CHO,$$

X représentant Cl ou Br,
et un produit de phosphoranylidénation d'un sel de phosphonium de formule

$$R^3CH_2P(R)_3X',$$

dans laquelle R est un groupe phényle ou un groupe tert.-butyle et X' est un atome d'halogène choisi dans la classe constituée par le chlore, le brome et l'iode.

2. Procédé selon la revendication 1, dans lequel $R^3$ représente un groupe $-CH_2-CH_3$.

3. Variante du procédé selon la revendication 1 ou 2, dans laquelle ledit 9-halo-(E)-2-nonen-1-al est obtenu par :
   - réaction d'une 8-halo-1-octyne avec un réactif de Grignard pour obtenir un halogénure de 8-halo-1-octyl-magnésium,
   - réaction dudit halogénure de 8-halo-1-octyl-magnésium avec un orthoformiate d'alkyle pour obtenir l'acétal correspondant, et
   - hydrogénation et hydrolyse du composé acétal pour obtenir ledit 9-halo-(E)-2-nonen-1-al.

4. Procédé de synthèse de composés phéromones sexuelles d'insectes ou de parfums, appartenant à la classe des composés (E,Z)-diéniques conjugués, en particulier ceux portant un motif terminal acétate, alcool ou aldéhyde, tels que l'acétate de E,Z-7,9-dodécadiényle, et le E,Z-10,12-hexadien-1-ol, comprenant une variante du procédé selon la revendication 1, dans lequel l'halogène est remplacé par un groupe acétate, hydroxy ou aldéhyde.

5. Composés alca-(E,Z)-diéniques conjugués, substitués par un halogène en position 1, répondant à la formule générale

$$R^3CH = (Z)CHCH = (E)CH(CH_2)_6X$$

dans laquelle X est un atome de chlore ou de brome, et $R^3$ est un groupe alkyle ayant 1 à 15 atomes de carbone, en particulier un groupe $CH_3-CH_2$.

**Patentansprüche**

1. Verfahren zur synthetischen Herstellung von in 1-Stellung durch Halogen substituierten, konjugierten Alka-(E,Z)-dienverbindungen der folgenden allgemeinen Formel:

$$R^3CH = (Z)CHCH = (E)CH(CH_2)_6 X,$$

worin X ein Chlor- oder Bromatom bedeutet und
$R^3$ ein Wasserstoffatom oder eine Alkylgruppe mit 1 bis 15 Kohlenstoffatome bedeutet,
worin man eine Wittigumsetzung eines 9-Halo-(E)-2-nonen-1-als der folgenden Formel

$$X-(CH_2)_6-CH = CH-CHO,$$

worin X für Cl oder Br steht,
mit einem Phosphoranylidenierungsprodukt eines Phosphoniumsalzes der folgenden Formel

$$R^3CH_2 P(R)_3 X',$$

worin R eine Phenylgruppe oder eine tertiäre Butylgruppe bedeutet und
X' ein Halogenatom bedeutet, das ausgewählt ist aus der Gruppe bestehend aus Chlor, Brom und Jod,
umsetzt.

2. Verfahren nach Anspruch 1, worin $R^3$ eine -$CH_2$-$CH_3$-Gruppe bedeutet.

3. Modifikation des Verfahrens nach Anspruch 1 oder 2, worin man das 9-Halo-(E)-2-nonen-1-al erhält, indem man
   - 8-Halo-1-octin mit einem Grignardreagenz zu einem 8-Halo-1-octin-1-yl-magnesiumhalogenid umsetzt,
   - dieses 8-Halo-1-octin-1-yl-magnesiumhalogenid mit einem Alkylorthoformiat zur Herstellung des entsprechenden Acetals umsetzt und
   - die Acetalverbindung zur Herstellung des 9-Halo-(E)-2-Nonen-1-als hydriert und hydrolysiert.

4. Verfahren zur synthetischen Herstellung von Sexpheromonen für Insekten oder von Duftstoffen der Klasse der konjugierten (E,Z)-Dienverbindungen, insbesondere mit einer Acetat-, Alkohol- oder Aldehydendeinheit, wie E,Z-7,9-Dodecadienylacetat und E,Z-10,12-Hexadecadien-1-ol unter Einschluß einer Modifikation des Verfahrens nach Anspruch 1, worin das Halogen durch eine Acetat-, Hydroxy- oder Aldehydgruppe ersetzt wird.

5. In 1-Stellung durch Halogen substituierte, konjugierte Alka-(E,Z)-dienverbindungen der folgenden allgemeinen Formel

$$R^3CH = (Z)CHCH = (E)CH(CH_2)_6 X,$$

worin X ein Chlor- oder Bromatom bedeutet und
$R^3$ eine Alkylgruppe mit 1 bis 15 Kohlenstoffatomen, insbesondere eine $CH_3$-$CH_2$-Gruppe bedeutet.